# EUROPEAN PATENT APPLICATION

(11) **EP 4 116 067 A1**
(43) Date of publication of application: **11.01.2023**
(21) Application number: 21305966.0
(22) Date of filing: 09.07.2021
(51) Int. Cl.: B29C 64/141, B33Y 70/00

(54) **STARCH BASED PRINTABLE MATERIALS**

(71) Applicant: ROQUETTE FRERES, 62136 Lestrem (FR)
(72) Inventor: LUI, Yuan-Siang, 138667 SINGAPORE (SG); CHOW, Keat-Theng, 138667 SINGAPORE (SG); GOKHALE, Rajeev, 138667 SINGAPORE (SG); HAEUSLER, Olaf, 59270 FLETRE (FR)
(74) Representative: Plasseraud IP

(57) **Abstract**

The instant invention relates to a printable material for 3D printing comprising a hydrolyzed and functionalized starch compound. It also relates to a process for 3D printing using the same. It also relates to products, in particular solid dosage forms, obtained thereof by 3D printing.

## Description

### Technical Field

The present invention relates to printable materials, in particular for use in the 3D printing of solid dosage forms.

### Background Art

3D printing (3DP), also known as additive manufacture (AM), is a process for constructing 3D physical objects from digital models through the successive layer-by-layer deposition of materials such as plastic, metal, ceramics, or even living cells.

Apart from many applications unrelated to healthcare, 3DP is currently used or under research for oral drugs, implantable drug delivery devices, tissue bioprinting, devices such as prosthesis and even food products.

When compared to traditional drug manufacturing, it was observed that 3DP is capable of producing reasonably priced, on-demand, patient tailored drugs and/or an increased product complexity. The shapes and internal structures of current drug products are limited by current technologies. When using additive manufacturing, these characteristics can be as complex as one can imagine, and this could prove useful especially for multiple drug release profiles embedded in the same dosage form. Some of the goals are minimising side effects, by achieving near-zero-order release by printing toroidal, cylindrical or perforated oral formulations or by using radial gradients of erosion or diffusion-controlling excipients. The ability to produce unique, individual or multi-drug and/or multi-dose formulations, can be the other important advantage of 3DP medicines, as demand for personalised medicine is increasing and becoming a megatrend.

Although research in the field started in the early 1990's, the FDA has approved only a single 3D printed drug since 2015: Spritam^{®}, an orodispersible dosage form obtained by binder jetting method 3DP, and having the following excipients: colloidal silicon dioxide, glycerin, mannitol, microcrystalline cellulose, polysorbate20, povidone, sucralose, butylated hydroxyanisole, natural and artificial spearmint flavor.

The 3DP methods that were adapted to handle pharmaceutical agents and biocompatible materials are: Extrusion printing (in particular Fused Deposition Modeling (FDM), pressure assisted microsyringe (PAM), and more recently, direct powder extrusion), Binder jetting (BJ), Material jetting (MJ), Stereolithography (SLA), Selective laser sintering (SLS). For more information about the 3DP classification, mention may be made of publication of reference lon-Bogdan Dumitrescu et al. "The age of pharmaceutical 3D printing technological and therapeutical implications of additive manufacturing. Farmacia, 2018, Vol. 66, 3 (365-389)", in particular Table I.

Extrusion printing, specifically FDM, is the most widely used method in the 3D printing field and is characterized by a pre-heated polymer filament or a semisolid being extruded through a nozzle in precise locations on a platform, using xyz positioning. Once the first layer cools down, the following one is applied. Depending on the shape of the printed product, a different material may be needed to provide structural support until the object cools down completely and solidifies, but afterwards it can be removed. PAM technology is similar, except that the printer is fed with non-melted, viscous materials. It is particularly useful for 3D Bioprinting. 3D Biopriting refers to methods that employ living cells. While the main advantage of biomedical manufactured scaffolds is the proliferation of living cells, from a pharmaceutical point of view they can also be a source for drug delivery, with antimicrobial drug-eluting implants as an example. Finally, mention can be made of direct powder extrusion, a technology that was recently developed. According to this technology, the printable powder is directly extruded thus avoiding the need for preparation of filaments.

The common 3D printing process chain includes the following step:
- Creation or adjustment of a CAD model, using specialised software, a 3D scan of an already existing object or medical imagery. The CAD file describes the geometry and size of the 3D structure.
- Conversion to one of the printer-ready STL or AMF file formats. These files describe the surface of the model in triangulated sections, depending on the surface curvature degree. Increasing the number of the triangulated sections will increase the resolution of the printed piece.
- Slicing the 3D model into layers of specified thickness using specialist software that can also determine the amount and position of support material, which prevents the collapsing of the printed object. The infill density of the printed object has to be specified as well - with 0% for a hollow structure and with 100% for a solid fill part.
- Transfer to and setting up of the files on the actual 3D printer.
- Layer-by-layer manufacturing of the 3D product.
- Occasionally, drying or removal and discarding of support material and post-processing are required.

Although 3DP represents a promising technology in the pharmaceutical field, there are still a limited variety of available, environmentally friendly, edible and printer-friendly materials, which is a bottleneck to the wide-scale adoption of 3D printing technologies. The most commonly used polymers (pharmaceutical grades) as drug carriers for the manufacture of dosage forms by FDM are polyvinylpyrrolidone (PVP), polyvinyl alcohol (PVA), and polylactic acid (PLA), which are all synthetic fossil oil-based materials.

In line with the current focus on the sustainable economy, the exploring of natural-derived and renewable biopolymers, instead of fossil oil-based materials, for the fabrication of various products has received tremendous attention.

A variety of natural derived biopolymers such as cellulose, hemicellulose, pectin, starch, alginate, agarose, and chitosan show potential to be utilized in 3D printing of edible dosage forms, owing to their nontoxicity, edibility, high abundancy, bioactivity (e.g. serve as dietary fibers), and long history of utilization in traditional food or medicine production.

However, such biomaterials need to be printable and need to allow the obtaining of a final product having satisfying properties (satisfying mechanical and/or biological and/or pharmacokinetic properties etc.). The problem is even more complex since according to the 3DP technology employed, the requirements for processability are different.

Overall, there is an unsatisfied need for printable materials derived from renewable resources, which would allow the obtaining of products, in particular of solid dosage forms, having all required properties.

### Objectives

Therefore, it is an object of the instant invention to provide printable materials and 3DP processes using ingredients obtained from renewable resources.

It is another object of the present invention to provide printable materials which are safe for both the operators and consumers.

It is another object of the present invention to provide printable materials which are easy to handle.

It is another object of the present invention to provide printable compositions and 3DP processes which allow the obtaining of products - in particular of solid dosage forms - having satisfying mechanical properties.

It is another object of the present invention to provide printable materials and 3DP processes allowing the obtaining of products containing active ingredients - in particular solid dosage forms - with satisfying release behavior and/or satisfying pharmacokinetic behavior, for instance with instant release properties, or with modified release properties, and/or providing enhanced water-solubility of the active ingredient.

### Presentation of the invention

The inventors found that specific starch compounds were very useful in 3DP processes. These starch compounds are hydrolyzed and chemically modified by the addition of functional groups.

As it will be apparent from the Examples hereinafter, the hydrolyzed and functionalized starch compound according to the invention is very versatile. It can be used in various 3DP technologies, in particular in 3DP technologies using extrusion, like in FDM or in PAM. The filaments obtained for FDM show good mechanical properties, especially when compared to starch compounds which are not functionalized, or when compared to standards PVA-based filaments.

The hydrolyzed and functionalized starch compounds according to the invention can be used in instant release dosage forms, and in modified or controlled release dosage forms. It can also be used for enhancing the water-solubility of water-insoluble active ingredients. This enhancement is higher than the one observed with dosage forms obtained by standard compression methods. In particular, the inventors showed that it was possible to obtain tablets by 3DP in which water-insoluble active ingredient solubility in water was ten times enhanced, as compared to tablets obtained by direct compression.

The hydrolyzed and functionalized starch compounds according to the disclosure is derived from renewable resources and can be formulated with ingredients derived from renewable resources as well.

This offers great and new perspectives not only for the development of environmentally friendly dosage forms by 3DP; but also for the development of dosage forms having pharmacokinetic properties that cannot be obtained otherwise, i.e. by using standard processes like tablet compression.

### Summary of the invention

The instant invention thus first relates to the use, for 3D printing, of a hydrolyzed and functionalized starch compound.

Preferably, said hydrolyzed and functionalized starch compound has a cold-water solubility equal to or higher than 30%, said percentage being expressed in dry weight of soluble starch with respect to the total weight of starch.

Preferably, said hydrolyzed and functionalized starch compound has a weight average molecular weight (Mw) lower than 10,000 kDa as determined by size-exclusion chromatography (HPSEC) attached to multiangle laser-light scattering (MALLS) and refractive index (RI) detectors.

Preferably, said hydrolyzed and functionalized starch compound has a viscosity lower than 10,000 mPa.s at 20°C, at a shear rate of 100 s⁻¹, as determined on an aqueous solution comprising 10% dry weight of said starch compound.

Preferably, said hydrolyzed and functionalized starch compound is selected from: a hydrolyzed starch compound functionalized with alkyl groups having 1 to 5 carbon atoms; an amphiphilic hydrolyzed and functionalized starch compound; or from a mixture thereof. A preferred hydrolyzed starch compound functionalized with alkyl groups having 1 to 5 carbon atoms is hydrolyzed hydroxypropyl starch. A preferred amphiphilic hydrolyzed and functionalized starch compound is octenylsuccinate starch.

Preferably, said hydrolyzed and functionalized starch compound is used in a printable material. Preferably, said printable material is a powder, a filament, or a gel mass.

Preferably, said use is for the 3D printing of a dosage form, preferably of a modified release dosage form or of an instant release dosage form.

Preferably, said use is for 3D printing by a process using extrusion, preferably by a process using hot melt extrusion (HME) or cold-extrusion.

The invention also relates to a process for the manufacture of a product by 3D printing, comprising the 3D printing of a printable material comprising a hydrolyzed and functionalized starch compound.

The invention also relates to a filament for 3D printing, comprising a hydrolyzed and functionalized starch compound.

### Brief Description of Drawings

**Fig. 1**

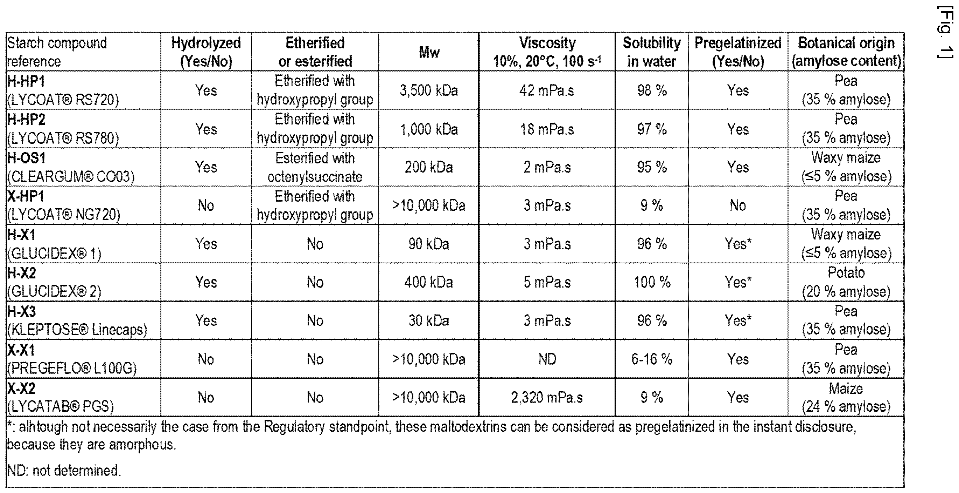

   [Fig. 1] is a table showing the characteristics of the starch compounds used in the Examples.
**Fig. 2**

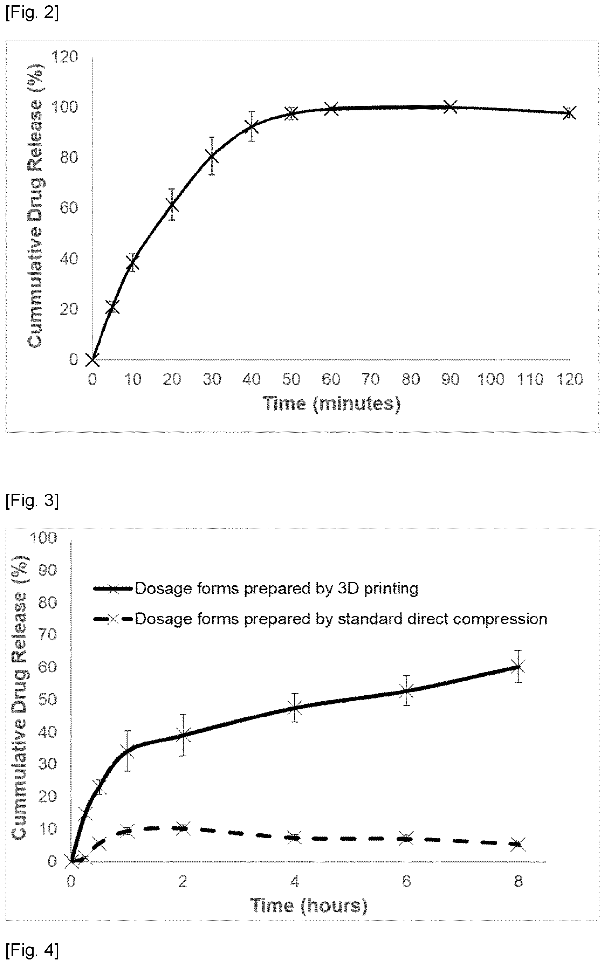

   [Fig. 2] shows the release profile of 3D printed dosage forms prepared by FDM technology according to the disclosure containing the water-soluble active ingredient diprophylline.
**Fig. 3**
   [Fig. 3] shows the release profile of 3D printed dosage forms prepared by FDM technology according to the disclosure containing water-insoluble active ingredient itraconazole and the release profile of printed dosage forms prepared by standard direct compression.
**Fig. 4**

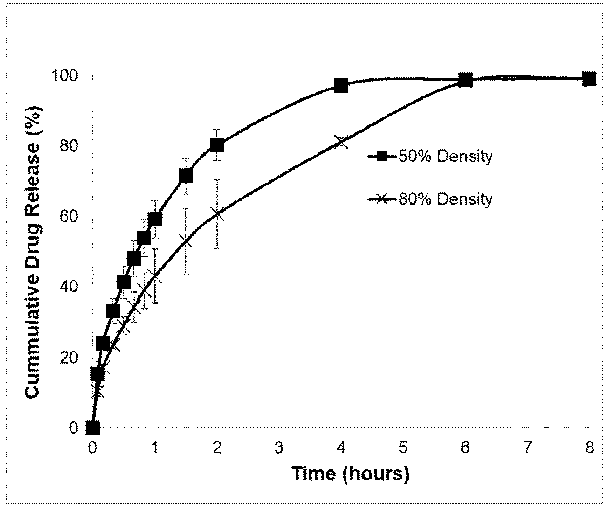

   [Fig. 4] shows the release profiles of 3D printed dosage forms prepared by PAM technology according to the disclosure, printed at 50% and 80% printing density, containing water soluble-active ingredient diprophylline.

### Description of Embodiments

The invention first relates to the use, for 3D printing (3DP), of a starch compound, wherein said starch compound:
- is hydrolyzed; and,
- is functionalized.

### Starch compounds:

The expression "starch compound" classically refers to a substance obtained from starch. It is reminded that the expression "starch" classically refers to the starch isolated from any suitable botanical source such as maize, tapioca barley by any technique well known to those skilled in the art. Isolated starch typically contains no more than 3% of impurities; said percentage being expressed in dry weight of impurities with respect to the total dry weight of isolated starch. These impurities typically comprise proteins, colloidal matters and fibrous residues. Suitable botanical source includes for instance legumes, cereals, and tubers. The starch compounds according to the disclosure can be derived from any suitable botanical source, including for example legumes (e.g. pea), cereals (e.g. maize, rice, wheat, oat), and tubers (e.g. potato, tapioca).

Hydrolyzed starch compounds according to the disclosure might be hydrolyzed by any suitable technique, for instance by acid treatment, heat treatment, enzymatic treatment, or a combination thereof. They can be dextrins, including maltodextrins or pyrodextrins, but excluding cyclodextrins. "Maltodextrins" classically refers to hydrolyzed starch compounds having a dextrose equivalent from 2 to 20. They are generally obtained by acid or enzymatic hydrolysis. "Pyrodextrins" are classically obtained from the action of high temperatures (generally of at least 100°C), combined with the action of an acid or alkaline or not ("British gums"), in the presence of small amounts of water. Such treatment classically leads to rebranchings and to the formation of so-called "atypical bounds" and pyrodextrins are therefore structurally different from the other hydrolyzed starch compounds, notably from maltodextrins. Preferably, the hydrolyzed starch compounds according to the disclosure are obtained by acid and/or enzymatic hydrolysis, for instance by β- or α-amylase hydrolysis, preferably by α-amylase hydrolysis.

Hydrolyzed starches have to be well differentiated from glucose, glucose syrups and cyclodextrins, the level of hydrolysis of which is too high to still be referred as "starches" as commonly understood by the person skilled in the art. In other words, glucose, glucose syrups and cyclodextrins, are not hydrolyzed starches within the meaning of the invention.

The functionalized starch compound according to the disclosure is hydrolyzed. This means that it has a reduced molecular weight, as compared to the native starch from which it is derived. Therefore, alternatively or complementarily, the hydrolyzed and functionalized compound according to the disclosure can be defined by its weight average molecular weight (Mw) which is preferably lower than 10,000 kDa as determined by size-exclusion chromatography (HPSEC) attached to multiangle laser-light scattering (MALLS) and refractive index (RI) detectors. Preferably, the Mw is determined by the following method: samples of starch compounds are diluted in a mixture of dimethylsulfoxide and sodium nitrate 0.1 M. Two columns are used having a porosity of 100 and 1000 Angstrom. Elution is performed in aqueous media at a flow rate of 0.5 mL/min. The system is maintained at a temperature of 40°C. This Mw can be for instance determined according to the detailed protocol as disclosed in the Examples. Preferably, the hydrolyzed and functionalized starch compound according to the disclosure has a weight average molecular weight (Mw) lower than 10,000 kDa, preferably equal to or lower than 9,000 kDa, still preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably lower than 4,000 kDa, still preferably equal to or lower than 3,500 kDa. It is preferably equal to or higher than 10 kDa, still preferably equal to or higher than 30 kDa, still preferably equal to or higher than 50 kDa, still preferably equal to or higher than 70 kDa, still preferably equal to or higher than 90 kDa, still preferably equal to or higher than 100 kDa, still preferably equal to or higher than 120 kDa, still preferably equal to or higher than 140 kDa, still preferably equal to or higher than 160 kDa, still preferably equal to or higher than 180 kDa, still preferably equal to or higher than 200 kDa. It is for example equal to about 3,500 kDa, or equal to about 1,000 Da, or equal to about 600 kDa, or equal to about 200 kDa.

Preferably, the hydrolyzed and functionalized starch compound according to the disclosure has a viscosity in water which is preferably lower than 10,000 mPa.s at 20°C, at a shear rate of 100 s⁻¹, as determined on an aqueous solution comprising 10% dry weight of said starch compound. Preferably, the viscosity is determined on a rheometer with a 5 cm 1° cone-plate geometry. This viscosity can be for instance determined according to the detailed protocol as disclosed in the Examples. Preferably, said viscosity is lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s. It is preferably equal to or higher than 1 mPa.s, still preferably equal to or higher than 2 mPa.s. it is for example equal to about 2 mPa.s, or equal to about 7 mPa.s, or equal to about 18 mPa.s, or equal to about 42 mPa.s.

The hydrolyzed starch compound according to the disclosure is also functionalized. In other words, the starch compound according to the disclosure has functional groups added to at least one of its hydroxyl functions. In other words the expression "functionalized" within the meaning of the invention excludes crosslinking. Preferably, the functionalized starch compound according to the disclosure is etherified and/or esterified. Preferably, said starch compound is functionalized, still preferably etherified and/or esterified, with groups having 1 to 20 carbon atoms, still preferably 1 to 15 carbon atoms, still preferably 2 to 12 carbon atoms, still preferably 3 to 12 carbon atoms.

The functionalized starch compound according to the disclosure might be etherified or esterified or both etherified and esterified. However, it is preferably either esterified or etherified. It can be substituted (i.e. functionalized) with groups of the same or different nature. However, it is preferably substituted with groups of same nature, for example substituted only with hydroxyropyl groups, or acetyl groups, or octenylsuccinyl groups.

Preferably, the functionalized starch compound according to the disclosure is selected from hydroxypropyl starch, acetyl starch, hydroxyethyl starches, octenylsuccinate starch or from a mixture thereof. It is preferably selected from hydroxypropyl starch, octenylsuccinate starch, or from a mixture thereof. Hydroxypropyl starches, acetyl starches, hydroxyethyl starches and octenylsuccinate starches are well known to those skilled in the art. Hydroxypropyl starch can be obtained for instance by etherification with propylene oxide. Acetyl starch can be obtained for instance by esterification with acetic anhydride. Octenylsuccinate starches can be obtained for instance by esterification with octenyl succinic anhydride. Hydroxyethyl starches starch can be obtained for instance by reaction of starch with ethylene oxide under alkaline conditions.

The hydrolyzed and functionalized starch compound according to the disclosure can be amphiphilic as it is the case for instance of octenylsuccinate starches.

Preferably, the hydrolyzed and functionalized starch compound according to the disclosure has a cold-water solubility equal to or higher than 30%, said percentage being expressed in dry weight of soluble starch with respect to the total weight of starch. Preferably, this cold-water solubility is equal to or higher than 40%, still preferably equal to or higher than 50%, still preferably equal to or higher than 60%, still preferably equal to or higher than 70%, still preferably equal to or higher than 80%, still preferably equal to or higher than 85%, still preferably equal to or higher than 90%, still preferably equal to or higher than 95%. It is for example equal to about 95%, or equal to about 97%, or equal to about 98%. This solubility can be determined for instance by putting 5 grams of the starch compound in 200 mL distilled water, at 20°C. The dry weight dissolved can be determined after centrifugation, and desiccation of the supernatant. This solubility can be for instance determined according to the detailed protocol as disclosed in the Examples. Preferred hydrolyzed and functionalized starch compounds are those having a cold-water solubility of at least 80% in dry weight of starch compound dissolved in water at room temperature (i.e. at about 20°C). They are called "cold-water soluble" hydrolyzed and functionalized starch compounds.

Cold water-soluble starch compound can be commonly obtained from insoluble starch or insoluble starch compound by cooking and/or hydrolyzing said insoluble starch or insoluble starch compound. Said cooking can typically be performed by heating a starch or starch compound suspension, in order for the insoluble granules to burst and solubilize. The functionalized starch compound according to the disclosure being hydrolyzed, it is sometimes cold-water soluble without the need of additional treatment, depending of course on the level of hydrolysis.

Preferably, the hydrolyzed and functionalized starch compound according to the disclosure is pregelatinized. Pregelatinization classically means that the starch compound particles no longer present birefringence (lack of crystalline phase) under an optical microscope in polarized light. Pregelatinization can be commonly obtained from birefringent starch or birefringent starch compound, *via* a thermal treatment (50-90°C in general, notably depending on the botanical origin of the starch) in presence of water (also often referred, like in the previous paragraphs, as "cooking") with further drying (after the cooking and/or concomitantly). Other chemical(s) might be used as process aid(s). Pregelatinization can be performed for example by drum-drying. In that case, the starch can be cooked before or during the drum-drying step. It can also be cooked and then spray-dried. It can also be obtained by extrusion. It is preferably performed by cooking and spray-drying.

In a preferred embodiment, the hydrolyzed and functionalized starch according to the disclosure is derived from starch having an amylose content by weight between 0 and 80%; said percentage being expressed in dry weight of amylose with respect to the total dry weight of starch. This amylose content can be determined by the person skilled in the art by way of potentiometric analysis of iodine absorbed by amylose to form a complex. It is preferably between 0 and 60%, still preferably between 0 and 50%, still preferably between 0 and 45%, still preferably between 0 and 40%.

The hydrolyzed and functionalized starch compound according to the disclosure might undergo other chemical and/or physical modification than the preferred ones exposed before, as long as it does not interfere with the desired properties, notably in term of safety and properties of the printable material and printed product obtained thereof. However, and because it appears that it is not necessary in the present invention, the hydrolyzed and functionalized starch compound according to the disclosure is preferably no further modified.

In a preferred embodiment, the hydrolyzed starch compound according to the disclosure is functionalized, still preferably etherified and/or esterified, with alkyl groups having 1 to 5 carbon atoms, preferably 2 to 4 carbon atoms, preferably 2 or 3 carbon atoms, preferably 3 carbon atoms. Preferably the alkyl group is an aliphatic alkyl group, still preferably a saturated aliphatic group.

Preferably, the hydrolyzed starch compound substituted with C₁-C₅ alkyl groups according to the disclosure is etherified.

Preferably, the hydrolyzed starch compound functionalized with C₁-C₅ alkyl groups according to the disclosure is hydrolyzed acetyl starch or hydrolyzed hydroxypropyl starch. In the case of hydrolyzed hydroxypropyl starch, the starch compound preferably has a content of hydroxypropyl groups between 0.5 and 10%; said percentage being expressed in dry weight of hydroxypropyl groups with respect to the total dry weight of hydrolyzed hydroxypropyl starch. The content of hydroxypropyl groups can be determined by the person skilled in the art for instance by Proton nuclear magnetic resonance (proton NMR), preferably according to a method complying with the European Pharmacopeia ("STARCH, HYDROXYPROPYL PREGELATINISED") as in force on March 1^{st}, 2021. It is preferably between 0.5 and 9%, still preferably between 0.5 and 8%, still preferably between 0.5 and 7%, for example equal to about 7%.

Preferably, the hydrolyzed starch compound functionalized with C₁-C₅ alkyl groups according to the disclosure is derived from legume and/or cereal starch, still preferably from pea or maize starch, still preferably from pea starch, still preferably from smooth pea starch. Preferably, the pea starch has an amylose content between 20 and 50%, still preferably between 30 and 45%, still preferably between 30 and 40%, for example equal to about 35%. Preferably, the maize starch has an amylose content greater than 5%, preferably between 10 and 40%, still preferably between 15 and 35%, still preferably between 20 and 30%, for example equal to about 25%.

Preferably, the hydrolyzed starch compound functionalized with C₁-C₅ alkyl groups according to the disclosure has a Mw lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably lower than 4,000 kDa, still preferably equal to or lower than 3,500 kDa. It is preferably equal to or higher than 100 kDa, still preferably equal to or higher than 200 kDa, still preferably equal to or higher than 400 kDa, still preferably equal to or higher than 600 kDa, still preferably equal to or higher than 800 kDa, still preferably equal to or higher than 900 kDa, still preferably equal to or higher than 1,000 kDa. It is for example equal to about 3,500 kDa, or equal to about 1,000 Da.

Preferably, the hydrolyzed starch compound functionalized with C₁-C₅ alkyl groups according to the disclosure has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s. It is preferably equal to or higher than 1 mPa.s, still preferably equal to or higher than 2 mPa.s, still preferably equal to or higher than 5 mPa.s, still preferably equal to or higher than 7 mPa.s, still preferably equal to or higher than 10 mPa.s, still preferably equal to or higher than 15 mPa.s. it is for example equal to 18 mPa.s, or equal to about 42 mPa.s..

Preferably, the hydrolyzed starch compound functionalized with C₁-C₅ alkyl groups according to the disclosure is hydrolyzed by acid-treatment.

Preferably, the hydrolyzed starch compound functionalized with C₁-C₅ alkyl groups according to the disclosure is cold water-soluble.

Preferably, the hydrolyzed starch compound functionalized with C₁-C₅ alkyl groups according to the disclosure is pregelatinized. It is preferably cooked. Still preferably, it is cooked and then spray-dried.

Preferably, the hydrolyzed starch compound functionalized with C₁-C₅ alkyl groups according to the disclosure is derived from a starch having an amylose content between 10 and 60%; said percentage being expressed in dry weight of amylose with respect to the total dry weight of starch. This amylose content is still preferably between 20 and 50%, still preferably between 30 and 45%, still preferably between 30 and 40%, for example equal to about 35%.

The hydrolyzed starch compound functionalized with C₁-C₅ alkyl groups according to the disclosure might undergo other chemical and/or physical modification than the preferred ones exposed before, as long as it does not interfere with the desired properties, notably in term of safety and properties of the printable material and printed product obtained thereof. However, and because it appears that it is not necessary in the present invention, the hydrolyzed starch compound functionalized with C₁-C₅ alkyl groups according to the disclosure is preferably no further modified.

Preferably, the hydrolyzed starch compound functionalized with C₁-C₅ alkyl groups according to the disclosure is a product of CAS N° 9049-76-7.

Preferably, the hydrolyzed starch compound functionalized with C₁-C₅ alkyl groups according to the disclosure is complying with European Pharmacopeia ("STARCH, HYDROXYPROPYL PREGELATINISED") as in force on March 1st, 2021

Hydrolyzed starch compound functionalized with C₁-C₅ alkyl groups particularly useful are commercially available. Mention can be made for instance of hydrolyzed hydroxypropyl starches LYCOAT^{®} RS 720 (CAS N°113894-92-1), or LYCOAT^{®} RS 780 (CAS N°113894-92-1) commercialized by the Applicant.

In another preferred embodiment, the hydrolyzed and functionalized starch compound according to the disclosure is amphiphilic. It is preferably functionalized, still preferably etherified and/or esterified, with groups having 8 to 20 carbon atoms, still preferably 9 to 15 carbon atoms, still preferably 11 to 13 carbon atoms, still preferably 12 carbon atoms. The added function is preferably anionic. It is preferably a sodium salt.

Preferably, the amphiphilic hydrolyzed starch compound according to the disclosure is esterified.

Preferably, the amphiphilic hydrolyzed and functionalized starch compound according to the disclosure is octenylsuccinate starch. In the case of hydrolyzed octenylsuccinate starch, the starch compound preferably has a content of octenylsuccinate groups between 0.5 and 5%; said percentage being expressed in dry weight of octenylsuccinyl groups with respect to the total dry weight of hydrolyzed octenylsuccinate starch. The content of octenylsuccinyl groups can be determined by the person skilled in the art for instance by a method complying with the FAO JECFA Food Starch, Modified, INS No. 1450 as in force on March 1^{st}, 2021. It is preferably lower or equal to 3%, preferably between 0.5 and 3%, for example equal to about 2%.

Preferably, the amphiphilic hydrolyzed and functionalized starch compound according to the disclosure is derived from maize starch, preferably from waxy maize starch.

Preferably, the amphiphilic hydrolyzed and functionalized starch compound according to the disclosure has Mw a lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably equal to or lower than 3,000 kDa, still preferably equal to or lower than 2,000 kDa, still preferably equal to or lower than 1,000 kDa, still preferably equal to or lower than 800 kDa, still preferably equal to or lower than 600 kDa, still preferably lower than 600 kDa, still preferably equal to or lower than 500 kDa, still preferably equal to or lower than 400 kDa, still preferably equal to or lower than 300 kDa. It is preferably equal to or higher than 10 kDa, still preferably equal to or higher than 30 kDa, still preferably equal to or higher than 50 kDa, still preferably equal to or higher than 70 kDa, still preferably equal to or higher than 90 kDa, still preferably equal to or higher than 100 kDa, still preferably equal to or higher than 120 kDa, still preferably equal to or higher than 140 kDa, still preferably equal to or higher than 160 kDa, still preferably equal to or higher than 180 kDa. It is for example equal to about 600kDa or equal to about 200 kDa.

Preferably, the amphiphilic hydrolyzed and functionalized starch compound according to the disclosure has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s, still preferably equal to or lower than 40 mPa.s, still preferably equal to or lower than 30 mPa.s, still preferably equal to or lower than 20 mPa.s, still preferably equal to or lower than 10 mPa.s, still preferably equal to or lower than 5 mPa.s It is preferably equal to or higher than 1 mPa.s, still preferably equal to or higher than 2 mPa.s. it is for example equal to 2 mPa.s, or equal to about 7 mPa.s.

Preferably, the amphiphilic hydrolyzed and functionalized starch compound according to the disclosure is hydrolyzed by enzymatic treatment, preferably with β-amylase and/or α-amylase, still preferably with α-amylase.

Preferably, the amphiphilic hydrolyzed and functionalized starch compound according to the disclosure is cold water-soluble. Preferably, the amphiphilic hydrolyzed and functionalized starch compound according to the disclosure is pregelatinized. It is preferably cooked. Still preferably, it is cooked and then spray-dried. Preferably, the hydrolysis is performed on the cooked starch. The slurry thus obtained is then preferably filtered before being spray-dried.

Preferably, the amphiphilic hydrolyzed and functionalized starch compound according to the disclosure is derived from a starch having an amylose content equal to or lower than 50%, said percentage being expressed in dry weight of amylose with respect to the total dry weight of starch. It is still preferably equal to or lower than 40%, still preferably equal to or lower than 30%, still preferably equal to or lower than 20%, still preferably equal to or lower than 10%, still preferably equal to or lower than 5%, still preferably equal to or lower than 3%, still preferably equal to or lower than 2%, still preferably equal to or lower than 1%, for example equal to about 0%. Still preferably, the hydrolyzed octenylsuccinate starch according to the disclosure is derived from waxy starch, still preferably from maize waxy starch. Such starches typically have amylose content of 0 to 5%.

The amphiphilic hydrolyzed and functionalized starch compound according to the disclosure might undergo other chemical and/or physical modification than the preferred ones exposed before, as long as it does not interfere with the desired properties, notably in term of safety and properties of the printable material and printed product obtained thereof. However, and because it appears that it is not necessary in the present invention, the amphiphilic hydrolyzed and functionalized starch compound according to the disclosure is preferably no further modified.

Preferably, the amphiphilic hydrolyzed and functionalized starch compound according to the disclosure is a product of CAS N° 66829-29-6.

Preferably, the amphiphilic hydrolyzed and functionalized starch compound according to the disclosure is complying with USP monograph as in force on March 1^{st}, 2021 and/or with the FAO JECFA Food Starch, Modified, INS No. 1450 as in force on March 1^{st}, 2021.

Amphiphilic hydrolyzed and functionalized starch compounds particularly useful are commercially available. Mention can be made for instance of octenylsuccinate starches CLEARGUM^{®} C003 (CAS N° 66829-29-6) or CLEARGUM^{®} C001 (CAS N°66829-29-6) commercialized by the Applicant.

Of course, the hydrolyzed and functionalized starch compounds according to the disclosure might be used alone or in combination. In particular, the two preferred starch compounds described before might be used alone or in combination.

### Printable materials:

The hydrolyzed and functionalized starch compounds according to the disclosure can be used in a printable material.

The invention thus also covers the use, for 3D printing, of a printable material comprising a hydrolyzed and functionalized starch compound.

The expression "printable material" classically refers to a material which can be used as such, optionally with the addition of a solvent, in a 3DP process. In other words, this is a ready-to-use material for feeding a 3D printer. The printable materials according to the disclosure can be in any suitable form. It can be in the liquid, semi-solid (e.g. gel mass) or solid (e.g. powder, filament) state. Preferably, the printable material according to the disclosure is a gel mass, a powder, or a filament, still preferably a filament. The printable powder can be directly used in 3DP (like in direct powder extrusion), or directly used in the preparation of printable filaments (like in FDM), or used for the preparation of a gel mass by the simple addition of a solvent, preferably water.

Preferably, the hydrolyzed and functionalized starch compound of the printable material according to the disclosure is as described before.

The hydrolyzed and functionalized starch compounds might be included alone or in combination in the printable material according to the disclosure.

Preferably, the hydrolyzed and functionalized starch compound of the printable material according to the disclosure is selected from: a hydrolyzed starch compound functionalized with C₁-C₅ alkyl groups, preferably as described before; an amphiphilic hydrolyzed and functionalized starch compound, preferably as described before; or from a mixture thereof. It is reminded here that the hydrolyzed starch compound functionalized with C₁-C₅ alkyl groups, preferably is hydrolyzed hydroxypropyl starch. It is reminded here that the amphiphilic hydrolyzed and functionalized starch compound, preferably is octenylsuccinate starch.

In a preferred embodiment, the amount of hydrolyzed and functionalized starch compound of the printable material according to the disclosure is equal to or higher than 1%, still preferably higher than 5%, still preferably higher than 10%, still preferably higher than 20%, still preferably higher than 30%, still preferably higher than 35%, still preferably equal to or higher than 40%; said percentages being expressed by weight with respect to the total weight of said printable material, the eventual solvents being excluded. It is preferably equal to or lower than 99%, still preferably lower than 90%, still preferably lower than 80%, still preferably lower than 70%, still preferably lower than 60%, still preferably lower than 55%, still preferably equal to or lower than 50%. It is for example equal to about 42% or equal to about 49%, or equal to about 50%.

In general, the printable materials according to the disclosure further includes other ingredients. Examples of such other ingredients are: active ingredients; sugars; plasticizers and sugar alcohols; colors; flavors; lubricants; other starches and starch compounds. Plasticizers and sugar alcohols according to the disclosure preferably are selected from glycerol, sorbitol, sorbitol anhydrides, maltitol, mannitol, xylitol, polyethylene glycol with a molecular weight of between 400 and 10 000 daltons, polyethylene glycol stearate, propylene glycol, triethyl citrate, acetyl triethyl citrate, tributyl citrate, polysorbate, acetylated monoglycerides, lactic acid esters, fatty acids and their salts or derivatives which are ethoxylated, such as, in particular, stearic acid, phthalates, ethyl sebacate, butyl sebacate, miglyol, glycerol triacetate, liquid paraffin, lecithin, carnauba wax, hydrogenated castor, urea, or from a mixture thereof. It is preferably selected from sugar alcohols, preferably from mannitol, sorbitol, or from a mixture thereof. Other examples of other ingredients are excipients classically used in 3D printing, in particular in FDM, like polylactic acid (PLA); hydroxypropylmethylcellulose (HPMC), cellulose acetate hydroxypropylcellulose (HPC), ethyl cellulose (EC), hydroxypropyl methylcellulose acetate succinate (HPMCAS), polyvinylpyrrolidone sigma (PVP), crospovidone, microcrystalline cellulose (MCC), Carbopol^{®} 794, polyethylene oxide , polyethylene glycol, Eudragit^{®}, polycaprolactone (PCL), triethyl citrate, tri-calcium phosphate, poly(vinyl alcohol) (PVA), Soluplus^{®}, Kollidon^{®}, Kollicoat^{®}, Polyox^{™}. Although the use of fossil-oil based excipients like PLA, PVP, crospovidone, Carbopol^{®} 794, polyethylene oxide, polyethylene glycol, Eudragit^{®}, PCL, triethyl citrate, tri-calcium phosphate, PVA, Soluplus^{®}, Kollidon^{®}, Kollicoat^{®}, or Polyox^{™} is preferably excluded.

In general, the amount of other ingredients (i.e. ingredients other than the hydrolyzed and functionalized starch compounds according to the disclosure) of the printable material according to the disclosure is equal to or higher than 1%, still preferably higher than 10%, still preferably higher than 20%, still preferably higher than 30%, still preferably higher than 40%, still preferably higher than 45%, still preferably equal to or higher than 50%; said percentages being expressed by weight with respect to the total weight of said printable material, the eventual solvents being excluded. It is preferably equal to or lower than 99%, still preferably lower than 95%, still preferably lower than 90%, still preferably lower than 80%, still preferably lower than 70%, still preferably lower than 65%, still preferably equal to or lower than 60%. It is for example equal to about 50%, or equal to about 51%, or equal to about 58%.

Preferably, the printable material according to the disclosure further comprises an active ingredient. The active ingredient according to the disclosure includes non-pharmaceutical and pharmaceutical agents. The expression "active" classically refers to any substance of pharmaceutical, veterinary, food, nutraceutical, cosmetic or agrochemical interest. Preferably, the active ingredient according to the disclosure is a pharmaceutical, nutraceutical, cosmetic or veterinary active ingredient. The active ingredient, in particular when it is a pharmaceutical active ingredient, may be chosen from so-called small molecules, but also from so-called "biological" active ingredients, as is the case for example of active substance based on or derived from proteins, nucleic acids - such as those derived from DNA or RNA - cells or viruses. Preferably, the active ingredient of the disclosure is selected from itraconazole, diprophylline, living cells, or from a mixture thereof.

The active ingredient according to the disclosure might be very soluble in water, freely soluble in water, soluble in water, sparingly soluble in water, slightly soluble in water, very slightly soluble in water or practically insoluble in water. This solubility in water is well defined for instance in 9th edition of The International Pharmacopeia (2019), Section "General Notice, Solubility". In the instant disclosure, the expression "water-insoluble" classically refers to active ingredients which are from sparingly soluble in water to practically insoluble in water. In the instant disclosure, the expression "water-soluble" classically refers to active ingredients which are from soluble in water to very soluble in water.

When the active ingredient according to the disclosure is water-insoluble it is preferably from slightly soluble in water to practically insoluble in water, still preferably very slightly soluble in water or practically insoluble in water, still preferably practically insoluble in water. When the active ingredient according to the disclosure is water-soluble it is preferably freely soluble in water or very soluble in water.

In general, the amount of active ingredient in the printable material according to the disclosure is equal to or higher than 1%, even equal to or higher than 2%, even equal to or higher than 5%, even equal to or higher than 10%; said percentages being expressed by weight with respect to the total weight of said printable material, the eventual solvents being excluded. It is in general equal to or lower than 99%, even equal to or lower than 90%, even equal to or lower than 70%, even equal to or lower than 50%, even equal to or lower than 40%, even equal to or lower than 30%, even equal to or lower than 20%. It is for example equal to about 10% or equal to about 20%.

Preferably, the printable material according to the disclosure is composed of:
- from 1 to 99% of hydrolyzed and functionalized starch compound according to the disclosure;
- from 1 to 99% of other ingredients;
- from 0 to 98% of solvent;
said percentages being expressed by weight with respect to the total weight of said printable material and their sum being equal to 100%.

Preferably, the amount of ingredients obtained from renewable sources in the printable material is equal to or higher than 50%, said percentage being expressed by weight with respect to the total weight of said printable material, the eventual solvents being excluded. Such ingredients obtained from renewable sources are in particular not obtained from fossil-oils. Still preferably, this amount is equal to or higher than 60%, still preferably equal to or higher than 70%, still preferably equal to or higher than 80%, still preferably equal to or higher than 90%, still preferably equal to 100%. Examples of ingredients which can be obtained from renewable sources are starch compounds, including the hydrolyzed and functionalized starch compounds according to the disclosure, cellulose derivatives like HPMC, HPC, EC, HPMCAS, or MCC, sugars and sugar alcohol. Example of ingredients which cannot be obtained from renewable sources are PLA, PVP, crospovidone, Carbopol^{®} 794, polyethylene oxide, polyethylene glycol, Eudragit^{®}, PCL, triethyl citrate, tri-calcium phosphate, PVA, Soluplus^{®}, Kollidon^{®}, Kollicoat^{®}, or Polyox^{™}.

Preferably, when an active ingredient is present, the excipients of the printable material according to the disclosure are composed of at least 50% of ingredients obtained from renewable sources, in particular not obtained from fossil-oils; said percentage being expressed by weight with respect to the total weight of excipients of said printable material, the eventual solvents being excluded. Still preferably, the excipients of the printable material according to the disclosure are composed of at least 60% by weight of ingredients obtained from renewable sources, still preferably of at least 70%, still preferably of at least 80%, still preferably of at least 90%, still preferably of 100%.

### Printed product:

The printable material according to the disclosure can be used for the 3DP of various products.

The printed product or product to be printed according to the disclosure typically is a solid or semi-solid product, preferably intended for oral administration, for instance a tablet, a gel, a film, a caplet, a softgel, a hard capsule. It can also be a drug loaded medical device. In a preferred embodiment, the printed product or product to be printed according to the disclosure is a solid product, typically a tablet. In a preferred embodiment, the printed product according to the disclosure is not a gel and/or not a film. The printed product or product to be printed according to the disclosure can be for instance of pharmaceutical, veterinary, nutraceutical, food, cosmetic, or agrochemical interest. It can be for humans or animals. It is preferably a pharmaceutical, veterinary, nutraceutical or cosmetic product. In a preferred embodiment, the printed product or product to be printed according to the disclosure is a dosage form (i.e. a product comprising an active ingredient), preferably a solid dosage form, preferably an oral solid dosage form, in particular a pharmaceutical oral solid dosage form.

In one preferred embodiment, the printed product according to the disclosure is an instant release dosage form. Preferably, the instant release property of the dosage form, is determined according to the guidance as provided by US FDA (Dissolution Testing and Acceptance Criteria for Immediate-Release Solid Oral Dosage Form Drug Products Containing High Solubility Drug Substances; Guidance for Industry ; August 2018, Biopharmaceutics). This release can in particular be determined using USP apparatus 2 (Paddle method), in 900mL of simulated gastric fluid media for 30 minutes. At least 80% by dry weight of the active ingredient should be released in 30 minutes or less.

In another preferred embodiment, the printed product according to the disclosure is a modified release dosage form or a controlled release dosage form. Preferably, the modified or controlled release property of the dosage form, is determined according to the same method as described just above for the instant release property, except that the duration is longer, for instance of at least 8 hours instead of 30 minutes. It is also possible to proceed according to the guidance as provided by the US FDA (General Methods; 32(2) Second Interim Revision Announcement: DISSOLUTION, <711> DISSOLUTION; 11/21/2016), using dissolution method A (pH transition method, simulated gastric fluid for 2 hours then change to simulated intestinal fluid for the remaining 10 hours). For a modified release dosage form, 80% by dry weight or less of the active ingredient should be released in 30 minutes, as opposed to instant release dosage forms. For controlled release dosage forms, 80% by dry weight or less of the active ingredient should dissolve in 8 hours. Of course, the active ingredient must still be released. Therefore, preferably, at least 20% by dry weight of the active ingredient should dissolve in 8 hours, preferably at least 30%, still preferably at least 40%, still preferably at least 50%, still preferably at least 60%.

The invention also covers a product obtained or obtainable by 3D printing, comprising a hydrolyzed and functionalized starch compound, said starch compound being preferably as described before in the specification. Preferably, the printed product of the instant disclosure has the composition as described before for the printable material, with the exception of the eventual added solvents or moisture of the ingredients used, which are ignored. Preferably, the printed product is as described above.

### 3D Printing process:

The hydrolyzed and functionalized starch compound or printable material according to the disclosure can be used for 3DP.

The 3D printing can be performed by any suitable method known to those skilled in the art. Preferably, 3D printing is carried out by a method selected from:
- extrusion, preferably fused deposition modeling (FDM), direct powder extrusion or pressure assisted microsyringe (PAM);
- binder jetting;
- material jetting;
- photopolymerisation, preferably stereolithography (SLA) or direct/digital light processing (DLP);
- powder bed fusion, preferably selective laser sintering (SLS).

Preferably, the 3D printing is performed by a process using extrusion. In one preferred embodiment, the 3D printing is performed by a process using hot melt extrusion (HME), preferably selected from FDM or direct powder extrusion, still preferably FDM. In another preferred embodiment, the 3D printing is performed by a process using cold-extrusion, preferably by PAM.

The invention also covers a process for the manufacture of a product by 3D printing, comprising the 3D printing of a printable material according to the disclosure. Preferably, the printed product or product to be printed is as described before. Preferably the 3D printing process is as described above.

### Use in instant release, and/or for formulating water-soluble ingredients, and/or in extrusion-based 3DP (FDM in particular):

Like mentioned before, the hydrolyzed functionalized starch compounds or printable materials according to the disclosure can advantageously be used for various purposes, and in various 3DP processes. As it is apparent from the Examples hereinafter, it can be used for the preparation of instant release dosage forms, and/or for formulating water-soluble active ingredients, and/or in 3DP by an extrusion method, preferably by HME, preferably by FDM or direct powder extrusion, still preferably by FDM.

Therefore, in a preferred embodiment, the hydrolyzed functionalized starch compound or printable material according to the disclosure is for the 3D printing of an instant release dosage form, and/or for the 3D printing of a dosage form comprising a water-soluble active ingredient, and/or for the 3D printing of dosage forms by an extrusion method, preferably by HME, in particular by FDM or direct powder extrusion, preferably by FDM.

Preferably, this hydrolyzed functionalized starch compound or printable material is for the 3D printing of a solid product, preferably a tablet. Preferably, this printable material is a powder or a filament, still preferably a filament. This product is preferably intended for oral administration. It is preferably a solid oral dosage form.

Preferably, the dosage form of this embodiment comprises at least an active ingredient. Preferably, this active ingredient is water-soluble, still preferably freely soluble in water or very soluble in water. It is preferably diprophylline, which is freely soluble in water.

Preferably, the hydrolyzed and functionalized starch compound used in this embodiment is functionalized with alkyl groups having 1 to 5 carbon atoms, preferably as described before. In particular, it is preferably hydrolyzed hydroxypropyl starch. Preferably, said hydrolyzed starch functionalized with alkyl groups having 1 to 5 carbon atoms has a Mw lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably lower than 4,000 kDa. It is preferably equal to or higher than 100 kDa, still preferably equal to or higher than 200 kDa, still preferably equal to or higher than 400 kDa, still preferably equal to or higher than 600 kDa, still preferably equal to or higher than 800 kDa, still preferably equal to or higher than 900 kDa, still preferably equal to or higher than 1,000 kDa, still preferably higher than 1,000 kDa, still preferably equal to or higher than 1,500 kDa, still preferably equal to or higher than 2,000 kDa, still preferably equal to or higher than 2,500 kDa, still preferably equal to or higher than 3,000 kDa. It is for example equal to about 3,500 kDa. Preferably, said hydrolyzed starch compound functionalized with alkyl groups having 1 to 5 carbon atoms has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s. It is preferably equal to or higher than 1 mPa.s, still preferably equal to or higher than 2 mPa.s, still preferably equal to or higher than 5 mPa.s, still preferably equal to or higher than 7 mPa.s, still preferably equal to or higher than 10 mPa.s, still preferably equal to or higher than 15 mPa.s, still preferably equal to or higher than 20 mPa.s, still preferably equal to or higher than 30 mPa.s, still preferably equal to or higher than 40 mPa.s. It is for example equal to about 42 mPa.s.

Preferably, the hydrolyzed and functionalized starch compounds are used in a printable material in this embodiment.

Preferably, the amount of hydrolyzed and functionalized starch compound of this printable material is equal to or higher than 1%, still preferably higher than 5%, still preferably higher than 10%, still preferably higher than 20%, still preferably higher than 24%, still preferably higher than 30%, still preferably higher than 35%, still preferably higher than 36%, still preferably higher than 40%; said percentages being expressed by weight with respect to the total weight of said printable material, the eventual solvents being excluded. It is preferably equal to or lower than 99%, still preferably lower than 90%, still preferably lower than 70%, still preferably lower than 60%, still preferably lower than 56%, still preferably lower than 55%, still preferably lower than 50%, still preferably lower than 45%, still preferably lower than 44%. It is for example equal to about 42%.

In general, the amount of active ingredient of this printable material is equal to or higher than 1%, even equal to or higher than 2%, even equal to or higher than 5%, even equal to or higher than 10%; said percentages being expressed by weight with respect to the total weight of said printable material, the eventual solvents being excluded. It is in general equal to or lower than 99%, even equal to or lower than 90%, even equal to or lower than 70%, even equal to or lower than 50%, even equal to or lower than 40%, even equal to or lower than 30%, for instance equal to about 20%.

Preferably this printable material further comprises a sugar alcohol or a plasticizer. Preferably, the plasticizer or sugar alcohol is selected from sorbitol, mannitol, or from a mixture thereof. It is preferably a mixture of mannitol and sorbitol. Preferably, the amount of sugar alcohol or plasticizer in this printable material is equal to or higher than 1%, still preferably higher than 5%, still preferably higher than 10%, still preferably higher than 15%, still preferably higher than 20%, still preferably higher than 22%, still preferably higher than 24%, still preferably higher than 30%, still preferably higher than 34%, still preferably higher than 35%; said percentages being expressed by weight with respect to the total weight of said printable material, the eventual solvents being excluded. It is preferably equal to or lower than 60%, still preferably lower than 55%, still preferably lower than 54%, still preferably lower than 50%, still preferably lower than 45%, still preferably lower than 42%, still preferably lower than 40%. It is for example equal to about 36%.

When a mixture of mannitol and sorbitol is used, the sorbitol : mannitol weight ratio is preferably higher than 1:1, still preferably equal to or higher than 2:1, still preferably equal to or higher than 3:1. It is preferably equal to or lower than 6:1, still preferably equal to or lower than 5:1. It is for example of about 4:1.

Preferably, this printable material further comprises an anti-sticking agent, preferably selected from meltable thermal lubricants for instance from metallic salts of fatty acids, fatty acids, fatty alcohol, fatty acid esters hydrocarbons, or form a mixture thereof. It is for example selected from glyceryl monostearate, magnesium stearate, calcium stearate, sodium stearate, hexadecane, octadecane, sodium stearyl fumarate, glyceryl behenate, acid stearic, or form a mixture thereof. It preferably comprises stearic acid. It is still preferably stearic acid alone. Preferably, the amount of anti-sticking agent is between 0.5 and 5%, preferably between 1 and 4%, preferably between 1 and 3%, for example equal to about 2%; said percentages being expressed by weight with respect to the total weight of said printable material, the eventual solvents being excluded.

Like mentioned before, the printable material according to the disclosure can be used as is in 3DP, eventually with the addition of a solvent. Preferably, the printable material of this embodiment is a powder or a filament. Therefore, this printable material preferably is devoid of added solvents, such as water.

Preferably, this printable material is composed of:
- from 1 to 97.5% of hydrolyzed and functionalized starch compound which is preferably functionalized with alkyl groups having 1 to 5 carbon atoms, in particular of hydrolyzed hydroxypropyl starch;
- from 1 to 97.5% of active ingredient;
- from 1 to 60% of plasticizer or sugar alcohol, preferably of a combination of mannitol and sorbitol;
- from 0.5 to 5% of anti-sticking agent;
- from 0 to 96.5% of other ingredients;
said percentages being expressed by weight with respect to the total weight of said printable material and their sum being equal to 100%.

Preferably, the amount of other ingredients of this printable material is lower than 90%, still preferably lower than 50%, still preferably lower than 30%, still preferably lower than 10%, still preferably lower than 5%, still preferably lower than 1%; said percentages being expressed by weight with respect to the total weight of said printable material, the eventual solvent being excluded. Still preferably, the printable material according to the disclosure is devoid of such other ingredients.

The invention also covers a printable material comprising a hydrolyzed and functionalized starch compound, like described in this embodiment. It also covers a dosage form, preferably an instant release dosage form, preferably comprising a water-soluble active ingredient, obtained or obtainable from this printable material, and/or having the composition of this printable material, with the exception of the eventual solvents which are ignored. It also covers a process for the manufacture of a product by 3D printing, comprising the 3D printing of this printable material. Preferably, the 3DP is performed by an extrusion method, preferably by FDM or direct powder extrusion, still preferably by FDM.

### Use in modified release and/or for solubility enhancement, and/or in extrusion-based 3DP(FMD in particular):

As it is apparent from the Examples hereinafter, the hydrolyzed functionalized starch compounds or printable materials according to the disclosure can also be used for the preparation of modified release dosage forms, preferably controlled release dosage forms, and/or for formulating water-insoluble active ingredients, and/or in 3DP by an extrusion method, preferably by HME, preferably by FDM or direct powder extrusion, still preferably by FDM.

Therefore, in another preferred embodiment, the hydrolyzed functionalized starch compound or printable material according to the disclosure is for the 3D printing of a modified release dosage form, preferably for the 3D printing of a controlled release dosage form, and/or for the 3D printing dosage form containing a water-insoluble active ingredient, and/or for the 3D printing of dosage forms by an extrusion method, preferably by HME, in particular by FDM or direct powder extrusion, preferably by FDM.

Preferably, this hydrolyzed functionalized starch compound or printable material is for the 3D printing of a solid product, preferably a tablet. Preferably, this printable material is a powder or a filament, still preferably a filament. This product is preferably intended for oral administration. It is preferably a solid oral dosage form.

Preferably, this dosage form comprises at least an active ingredient, which is preferably water-insoluble, still preferably from slightly soluble in water to practically insoluble in water, still preferably very slightly soluble in water or practically insoluble in water, still preferably practically insoluble in water. It is preferably itraconazole, which is practically insoluble in water.

Preferably, the hydrolyzed and functionalized starch compound used in this embodiment is selected from: hydrolyzed starch compound functionalized with C₁-C₅ alkyl groups, preferably as described before; amphiphilic hydrolyzed and functionalized starch compound, preferably as described before; or from a mixture thereof. It is still preferably a mixture thereof. It is reminded here that the hydrolyzed starch compound functionalized with C₁-C₅ alkyl groups, preferably is hydrolyzed hydroxypropyl starch. It is reminded here that the amphiphilic hydrolyzed and functionalized starch compound, preferably is hydrolyzed octenylsuccinate starch.

Preferably, the hydrolyzed starch compound functionalized with C₁-C₅ alkyl groups used in this embodiment has a Mw lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably equal to or lower than 3,500 kDa, still preferably lower than 3,500 kDa, still preferably equal to or lower than 3,000 kDa, still preferably equal to or lower than 2,500 kDa, still preferably equal to or lower than 2,000 kDa, still preferably equal to or lower than 1,500 kDa. It is preferably equal to or higher than 100 kDa, still preferably equal to or higher than 200 kDa, still preferably equal to or higher than 400 kDa, still preferably equal to or higher than 600 kDa, still preferably equal to or higher than 800 kDa, still preferably equal to or higher than 900 kDa. It is for example equal to about 1,000 Da. Preferably, said hydrolyzed starch compound functionalized with C₁-C₅ alkyl groups has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s, still preferably equal to or lower than 40 mPa.s, still preferably equal to or lower than 30 mPa.s, still preferably equal to or lower than 20 mPa.s. It is preferably equal to or higher than 1 mPa.s, still preferably equal to or higher than 2 mPa.s, still preferably equal to or higher than 5 mPa.s, still preferably equal to or higher than 7 mPa.s, still preferably equal to or higher than 10 mPa.s, still preferably equal to or higher than 15 mPa.s. it is for example equal to 18 mPa.s.

Preferably, the amphiphilic hydrolyzed and functionalized starch compound used in this embodiment has a Mw lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably equal to or lower than 3,000 kDa, still preferably equal to or lower than 2,000 kDa, still preferably equal to or lower than 1,000 kDa, still preferably equal to or lower than 800 kDa, still preferably equal to or lower than 600 kDa, still preferably lower than 600 kDa, still preferably equal to or lower than 500 kDa, still preferably equal to or lower than 400 kDa, still preferably equal to or lower than 300 kDa. It is preferably equal to or higher than 10 kDa, still preferably equal to or higher than 30 kDa, still preferably equal to or higher than 50 kDa, still preferably equal to or higher than 70 kDa, still preferably equal to or higher than 90 kDa, still preferably equal to or higher than 100 kDa, still preferably equal to or higher than 120 kDa, still preferably equal to or higher than 140 kDa, still preferably equal to or higher than 160 kDa, still preferably equal to or higher than 180 kDa. It is for example equal to about 200 kDa. Preferably, said amphiphilic hydrolyzed and functionalized starch compound has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s, still preferably equal to or lower than 40 mPa.s, still preferably equal to or lower than 30 mPa.s, still preferably equal to or lower than 20 mPa.s, still preferably equal to or lower than 10 mPa.s, still preferably equal to or lower than 5 mPa.s It is preferably equal to or higher than 1 mPa.s, still preferably equal to or higher than 2 mPa.s. it is for example equal to 2 mPa.s.

Preferably, the hydrolyzed and functionalized starch compounds is are used in a printable material in this embodiment.

Preferably, the amount of hydrolyzed and functionalized starch compound of this printable material is equal to or higher than 1%, still preferably higher than 5%, still preferably higher than 10% still preferably higher than 20%, still preferably higher than 30%, still preferably higher or equal to 35%, still preferably higher than 35%, still preferably higher than 40%, still preferably higher than 45% ; said percentages being expressed by weight with respect to the total weight of said printable material, the eventual solvents being excluded. It is preferably equal to or lower than 99%, still preferably lower than 90%, still preferably lower than 70%, still preferably lower than 60%, still preferably lower than 55%. It is for example equal to about 50 %.

When hydrolyzed starch compound functionalized with C₁-C₅ alkyl groups is used in the printable material, the amount of said starch compound is preferably equal to or higher than 1%, still preferably higher than 5%, still preferably higher than 10%, still preferably higher than 12%, still preferably higher than 15%, still preferably higher than 17%, still preferably equal to or higher than 20%, still preferably higher than 20%, still preferably equal to or higher than 25%, still preferably higher than 25%; said percentages being expressed by weight with respect to the total weight of said printable material, the eventual solvents being excluded. It is preferably equal to or lower than 99%, still preferably lower than 90%, still preferably lower than 70%, still preferably lower than 60%, still preferably lower than 50%, still preferably lower than 40%, still preferably lower than 38%, still preferably lower than 35%, still preferably lower than 33%, still preferably equal to or lower than 30%, still preferably lower than 30%. It is for example equal to about 26%.

When amphiphilic hydrolyzed and functionalized starch compound is used in the printable material, the amount of said starch compound is preferably equal to or higher than 1%, still preferably higher than 5%, still preferably higher than 10%, still preferably higher than 12%, still preferably equal to or higher than 15%, still preferably higher than 15%; said percentages being expressed by weight with respect to the total weight of said printable material, the eventual solvents being excluded. It is preferably equal to or lower than 99%, still preferably lower than 90%, still preferably lower than 70%, still preferably lower than 60%, still preferably lower than 50%, still preferably lower than 40%, still preferably lower than 38%, still preferably lower than 35%, still preferably lower than 33%, still preferably equal to or lower than 30%, still preferably lower than 30%. It is for example equal to about 24%.

In general, the amount of active ingredient in this printable material is equal to or higher than 1%, even equal to or higher than 2%, even equal to or higher than 5%; said percentages being expressed by weight with respect to the total weight of said printable material, the eventual solvents being excluded. It is in general equal to or lower than 99%, even equal to or lower than 90%, even equal to or lower than 70%, even equal to or lower than 50%, even equal to or lower than 40%, even equal to or lower than 30%, even equal to or lower than 20%, even equal to or lower than 15%. It is for example of about 10%.

Preferably, this printable material further comprises a sugar alcohol or a plasticizer. Preferably, the sugar alcohol or plasticizer is selected from sorbitol, mannitol, or from a mixture thereof. Preferably, the amount of sugar alcohol or plasticizer in this printable material is equal to or higher than 1%, still preferably higher than 5%, still preferably higher than 10%, still preferably higher than 20%, still preferably higher than 25%, still preferably higher than 30%, still preferably equal to or higher than 35%, still preferably higher than 35%; said percentages being expressed by weight with respect to the total weight of said printable material, the eventual solvents being excluded. It is preferably equal to or lower than 90%, still preferably lower than 80%, still preferably lower than 70%, still preferably lower than 60%, still preferably lower than 50%, still preferably equal to or lower than 45%, still preferably lower than 45%. It is for example equal to about 40%.

Like mentioned before, the printable material according to the disclosure can be used as is in 3DP, eventually with the addition of a solvent. Preferably, the printable material of this embodiment is a powder or a filament. Therefore, this printable material preferably is devoid of added solvents, such as water.

Preferably, this printable material is composed of:
- from 1 to 98% of hydrolyzed and functionalized starch compound according to the disclosure, in particular of a combination of hydrolyzed hydroxypropyl starch and hydrolyzed octenylsuccinate starch;
- from 1 to 98% of active ingredient;
- from 1 to 90% of plasticizer or sugar alcohol, preferably of sorbitol;
- from 0 to 97% of other ingredients;
said percentages being expressed by weight with respect to the total weight of said printable material and their sum being equal to 100%.

Preferably, the amount of other ingredients of this printable material is lower than 90%, still preferably lower than 50%, still preferably lower than 30%, still preferably lower than 10%, still preferably lower than 5%, still preferably lower than 1%; said percentages being expressed by weight with respect to the total weight of said printable material, the eventual solvent being excluded. Still preferably, the printable material according to the disclosure is devoid of such other ingredients.

The invention also covers a printable material comprising a hydrolyzed and functionalized starch compound, like described in this embodiment. It also covers a dosage form, preferably a modified release dosage form, still preferably a controlled release dosage form, and/or a solid form comprising a water-insoluble active ingredient, obtained or obtainable from this printable material, and/or having the composition of this printable material, with the exception of the eventual solvents which are ignored. It also covers a process for the manufacture of a product by 3D printing, comprising the 3D printing of this printable material. Preferably, the 3DP is performed by an extrusion method, preferably by FDM or direct powder extrusion, still preferably by FDM.

### Use in modified release of active ingredients, and/or for formulating water-soluble active ingredients, and/or in extrusion-based 3DP (PAM in particular):

As it is apparent from the Examples hereinafter, the hydrolyzed functionalized starch compounds or printable materials according to the disclosure can be used for the preparation of modified release dosage forms, and/or for formulating water-soluble active ingredients, and/or in 3DP by an extrusion method, preferably by cold-extrusion, preferably by PAM.

Therefore, in a preferred embodiment, the hydrolyzed functionalized starch compound or printable material according to the disclosure is for the 3D printing of a modified release dosage form, and/or for the 3D printing of a dosage form comprising a water-soluble active-ingredient, and/or for the 3D printing of dosage forms by an extrusion method, preferably by cold-extrusion, in particular by PAM, and/or for 3D bioprinting.

Preferably, this hydrolyzed functionalized starch compound or printable material is for the 3D printing of a solid product, preferably a tablet. Preferably, this printable material is a gel mass. This product is preferably intended for oral administration. It is preferably a solid oral dosage form.

Preferably, this dosage form comprises at least an active ingredient.

In one preferred embodiment, the active ingredient is water-soluble, still preferably freely soluble in water or very soluble in water. It is preferably diprophylline, which is freely soluble in water. In another preferred embodiment, the active ingredient is cells.

Preferably, the hydrolyzed and functionalized starch compound used in this embodiment is selected from: hydrolyzed starch compound functionalized with C₁-C₅ alkyl groups, preferably as described before; amphiphilic hydrolyzed and functionalized starch compound, preferably as described before; or from a mixture thereof. It is still preferably a mixture thereof. It is reminded here that the hydrolyzed starch compound functionalized with C₁-C₅ alkyl groups, preferably is hydrolyzed hydroxypropyl starch. It is reminded here that the amphiphilic hydrolyzed and functionalized starch compound, preferably is octenylsuccinate starch.

Preferably, the hydrolyzed starch compound functionalized with C₁-C₅ alkyl groups used in this embodiment has a Mw lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably equal to or lower than 3,500 kDa, still preferably lower than 3,500 kDa, still preferably equal to or lower than 3,000 kDa, still preferably equal to or lower than 2,500 kDa, still preferably equal to or lower than 2,000 kDa, still preferably equal to or lower than 1,500 kDa. It is preferably equal to or higher than 100 kDa, still preferably equal to or higher than 200 kDa, still preferably equal to or higher than 400 kDa, still preferably equal to or higher than 600 kDa, still preferably equal to or higher than 800 kDa, still preferably equal to or higher than 900 kDa. It is for example equal to about 1,000 Da. Preferably, said hydrolyzed starch compound functionalized with C₁-C₅ alkyl groups has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s, still preferably equal to or lower than 40 mPa.s, still preferably equal to or lower than 30 mPa.s, still preferably equal to or lower than 20 mPa.s. It is preferably equal to or higher than 1 mPa.s, still preferably equal to or higher than 2 mPa.s, still preferably equal to or higher than 5 mPa.s, still preferably equal to or higher than 7 mPa.s, still preferably equal to or higher than 10 mPa.s, still preferably equal to or higher than 15 mPa.s. it is for example equal to 18 mPa.s.

Preferably, the amphiphilic hydrolyzed and functionalized starch compound used in this embodiment has a Mw lower than 9,000 kDa as determined by size-exclusion chromatography (HPSEC) attached to multiangle laser-light scattering (MALLS) and refractive index (RI) detectors. It is preferably equal to or lower than 8,000 kDa, still preferably equal to or lower than 7,000 kDa, still preferably equal to or lower than 6,000 kDa, still preferably equal to or lower than 5,000 kDa, still preferably equal to or lower than 4,000 kDa, still preferably equal to or lower than 3,000 kDa, still preferably equal to or lower than 2,000 kDa, still preferably equal to or lower than 1,000 kDa, still preferably equal to or lower than 800 kDa, still preferably equal to or lower than 600 kDa, still preferably lower than 600 kDa, still preferably equal to or lower than 500 kDa, still preferably equal to or lower than 400 kDa, still preferably equal to or lower than 300 kDa. It is preferably equal to or higher than 10 kDa, still preferably equal to or higher than 30 kDa, still preferably equal to or higher than 50 kDa, still preferably equal to or higher than 70 kDa, still preferably equal to or higher than 90 kDa, still preferably equal to or higher than 100 kDa, still preferably equal to or higher than 120 kDa, still preferably equal to or higher than 140 kDa, still preferably equal to or higher than 160 kDa, still preferably equal to or higher than 180 kDa. It is for example equal to about 200 kDa. Preferably, said amphiphilic hydrolyzed and functionalized starch compound has a viscosity lower than 8,000 mPa.s at 20°C, at a shear rate of 100 s⁻¹, as determined on an aqueous solution comprising 10% dry weight of said starch compound. It is preferably lower than or equal to 8,000 mPa.s, still preferably equal to or lower than 6,000 mPa.s, still preferably equal to or lower than 4,000 mPa.s, still preferably equal to or lower than 2,000 mPa.s, still preferably equal to or lower than 1,000 mPa.s, still preferably equal to or lower than 500 mPa.s, still preferably equal to or lower than 300 mPa.s, still preferably equal to or lower than 200 mPa.s, still preferably equal to or lower than 100 mPa.s, still preferably equal to or lower than 50 mPa.s, still preferably equal to or lower than 40 mPa.s, still preferably equal to or lower than 30 mPa.s, still preferably equal to or lower than 20 mPa.s, still preferably equal to or lower than 10 mPa.s, still preferably equal to or lower than 5 mPa.s It is preferably equal to or higher than 1 mPa.s, still preferably equal to or higher than 2 mPa.s. it is for example equal to 2 mPa.s.

Preferably, the hydrolyzed and functionalized starch compounds are used in a printable material in this embodiment.

Preferably, the amount of hydrolyzed and functionalized starch compound of this printable material is equal to or higher than 1%, still preferably higher than 5%, still preferably higher than 10%, still preferably higher than 20%, still preferably higher than 30%, still preferably higher than 40%, still preferably higher than 45%; said percentages being expressed by weight with respect to the total weight of said printable material, the eventual solvent being excluded. It is preferably equal to or lower than 99%, still preferably lower than 90%, still preferably lower than 80%, still preferably lower than 70%, still preferably lower than 60%, still preferably lower than 55%, still preferably lower than 50%. It is for example equal to about 49%.

When hydrolyzed starch compound functionalized with C₁-C₅ alkyl groups is used in the printable material, the amount of said starch compound is preferably equal to or higher than 1%, still preferably higher than 5%, still preferably higher than 10%; said percentages being expressed by weight with respect to the total weight of said printable material, the eventual solvent being excluded. It is in preferably equal to or lower than 70%, still preferably lower than 60%, still preferably lower than 50%, still preferably lower than 40%, still preferably lower than 30%, still preferably lower than 20%, still preferably lower than 15%. It is for example equal to about 12%.

When amphiphilic hydrolyzed and functionalized starch compound is used in the printable material, the amount of said starch compound is preferably equal to or higher than 1%, still preferably higher than 5%, still preferably higher than 10%, still preferably higher than 20%, still preferably higher than 30%, still preferably higher than 35%; said percentages being expressed by weight with respect to the total weight of said printable material, the eventual solvent being excluded. It is preferably equal to or lower than 70%, still preferably lower than 60%, still preferably lower than 50%, still preferably lower than 45%, still preferably lower than 40%. It is for example equal to about 37%.

In general, the amount of active ingredient of this printable material is equal to or higher than 1%, even equal to or higher than 2%, even equal to or higher than 5%; said percentages being expressed by weight with respect to the total weight of said printable material, the eventual solvents being excluded. It is in general equal to or lower than 99%, even equal to or lower than 90%, even equal to or lower than 70%, even equal to or lower than 50%, even equal to or lower than 40%, even equal to or lower than 30%, even equal to or lower than 20%, even equal to or lower than 15%. It is for example of about 10%.

Preferably, said printable material further comprises an additional starch compound selected from a cold water-soluble and/or pregelatinized cross-linked starch; a cold water-soluble and/or pregelatinized waxy starch; or from a mixture thereof. Without being bond by any theory, the inventors believe that such starch are improving the control release properties of the dosage form when such release property is desired.

It is preferably both cold water-soluble and pregelatinized.

Preferably, when the additional starch compound is cold water-soluble and/or pregelatinized cross-linked starch, it is derived from potato starch. It is preferably cooked and drum-dried.

Preferably, when the additional starch compound is cold water-soluble and/or pregelatinized cross-linked starch, it is cross-linked with sodium trimetaphosphate.

Preferably, the cold water-soluble and/or pregelatinized cross-linked starch according to the disclosure is a product of CAS N° 55963-33-2.

Cold water-soluble and/or pregelatinized cross-linked starches particularly useful are commercially available. Mention can be made for instance of PREGEFLO^{®} PI10 commercialized by the Applicant.

Preferably, when the additional starch compound is cold water-soluble and/or pregelatinized waxy starch, it is derived from maize waxy starch.

Preferably, the amount of said additional starch compound of this printable material is equal to or higher than 1%, still preferably higher than 2%, still preferably higher than 5%, still preferably higher than 10%, still preferably higher than 15%; said percentages being expressed by weight with respect to the total weight of said printable material, the eventual solvent being excluded. It is preferably equal to or lower than 99%, still preferably lower than 90%, still preferably lower than 70%, still preferably lower than 50%, still preferably lower than 40%, still preferably lower than 30%, still preferably lower than 25%. It is for example equal to about 20%.

Preferably, this printable material further comprises microcrystalline cellulose (MMC).

Preferably, said MCC as an average particle size equal to or lower than 400 µm, still preferably equal to or lower than 200 µm, still preferably equal to or lower than 100 µm, still preferably equal to or lower than 80 µm, still preferably equal to or lower than 60 µm, for example equal to about 50 µm.

Preferably, the amount of said microcrystalline cellulose in this printable material is equal to or higher than 1%, still preferably higher than 2%, still preferably higher than 5%, still preferably higher than 10%, still preferably higher than 15%; said percentages being expressed by weight with respect to the total weight of said printable material, the eventual solvent being excluded. It is preferably equal to or lower than 99%, still preferably lower than 90%, still preferably lower than 80%, still preferably lower than 70%, still preferably lower than 60%, still preferably lower than 50%, still preferably lower than 40%, still preferably lower than 30%, still preferably lower than 25%. It is for example equal to about 20%.

Like mentioned before, the printable material according to the disclosure can be used as is in 3DP, eventually with the addition of a solvent. Preferably, the printable material of this embodiment is a gel-mass. In that case it preferably further contains a solvent, preferably a polar solvent, preferably water.

The amount of solvent of this printable material preferably is equal to or higher than 10%, still preferably higher than 20%, still preferably higher than 30%, still preferably equal to or higher than 40%; said percentage being expressed by weight with respect to the total weight of said gel-mass. It is preferably equal to or lower than 80%, still preferably lower than 70%, still preferably lower than 60%, still preferably lower than 50%. It is for example equal to about 45%.

Preferably, this printable material is composed of:
(a) from 1 to 100% of ingredients other than solvents which are composed of:
   - from 1 to 97% of hydrolyzed and functionalized starch compound according to the disclosure, in particular of a combination of hydrolyzed hydroxypropyl starch and hydrolyzed octenylsuccinate starch;
   - from 1 to 97% of active ingredient;
   - from 1 to 97% of additional starch compound;
   - from 1 to 97% of microcrystalline cellulose;
   - from 0 to 96% of other ingredients;
(b) from 0 to 99% of a solvent, preferably of water;
   said percentages (a) and (b) being expressed by weight with respect to the total weight of said printable material and their sum being equal to 100%.

Preferably, the amount of other ingredients of this printable material is lower than 90%, still preferably lower than 50%, still preferably lower than 30%, still preferably lower than 10%, still preferably lower than 5%, still preferably lower than 1%; said percentages being expressed by weight with respect to the total weight of said printable material, the eventual solvent being excluded. Still preferably, the printable material according to the disclosure is devoid of such other ingredients.

The invention also covers a printable material comprising a hydrolyzed and functionalized starch compound, like described in this embodiment. It also covers a dosage form, preferably a modified release dosage form, preferably comprising a water-soluble active ingredient, obtained or obtainable from this printable material, and/or having the composition of this printable material, with the exception of the eventual solvents which are ignored. It also covers a dosage form obtained or obtainable from this printable material, and/or having the composition of this printable material, with the exception of the eventual solvents which are ignored, wherein said dosage form further comprises cells. It also covers a process for the manufacture of a product by 3D printing or by 3D bioprinting, comprising the 3D printing of this printable material. Preferably, the 3DP is performed by an extrusion method, preferably by cold-extrusion, preferably by PAM.

In the instant disclosure, the expression "between" classically includes the recited upper and lower limits.

In the instant disclosure, the amounts of ingredients are generally expressed in percentages by weight. Unless otherwise specified these weights are amounts of ingredients in their original form, which is generally in powder form. These powdery ingredients generally include small amounts of water (also referred as %moisture or as "loss on drying"). With that respect: starch compounds according to the disclosure generally contain no more than 15% by weight of water, generally 3 to 12%; sugar alcohol like mannitol and sorbitol generally contain no more than 1% water. This means that when it is for example referred to 10% by weight of a starch compound, this generally corresponds to 8.8 to 9.7% in dry weight (i.e. anhydrous weight).

By opposition, in the instant disclosure, when it is referred to dry weight, this well refers to anhydrous weights.

Other characteristics and advantages of the present invention will emerge clearly on reading the examples given hereinafter, which illustrate the invention without however limiting it.

### Examples

### A. Materials

### A.1. Starch compounds used and characterization thereof.

Mw measurement. The Mw was determined by size-exclusion chromatography (HPSEC) attached to multiangle laser-light scattering (MALLS) and refractive index (RI) detectors, by the following method: samples of starch compounds were diluted in a mixture of dimethylsulfoxide and sodium nitrate 0.1 M. Two columns were used (SUPREMA) which had a porosity of 100 and 1000 Angstrom. Elution was performed in aqueous media at a flow rate of 0.5 mL/min. The system was maintained at a temperature of 40°C.

Viscosity measurement. Measurements were performed on a rheometer (Physica MCR301, Anton Paar) with a 5 cm 1° cone-plate geometry (CP50). The temperature was regulated by way of Peltier. The measurements were performed at 20°C on an aqueous solution comprising 10% dry starch compound as follow: 1 minute equilibrium at 20°C; shear rates from 0.006 to 1,000 s⁻¹(log) applied in 3 minutes at 20°C.

Solubility measurement. In a 250 ml beaker, 200 ml of distilled water were added. 5 grams weigh of starch compound were added and the mixture was homogenized by magnetic agitation for 15 minutes. The resulting solution / suspension was centrifuged 10 minutes at 4,000 rpm. 25 mL of the supernatant were taken out and introduced into a crystallizer and place into an oven at 60°C, until the water evaporates. It was then placed into an oven at 103+C ± 2°C for 1 hour. The residue was placed into a desiccator to cold down at room temperature and then weighted for calculating the solubility in dry weight.

The characteristics of the various starchy compounds used are presented in Figure 1.

### A.2. Other ingredients used

The following other ingredients were used: sorbitol (NEOSORB^{®} 100C, ROQUETTE), mannitol (PEARLITOL^{®} 100SD, ROQUETTE); stearic acid (Stearic acid 50, SIGMA-ALDRICH), diprophylline (Shanghai Star), itraconazole (Shanghai Star), crosslinked cold water-soluble potato starch (PREGEFLO^{®} PI10, ROQUETTE), microcrystalline cellulose with an average particle size of about 50 µm (MCC101, ROQUETTE).

### B. Preparation of instant release tablets comprising a water-soluble active ingredient by FDM

In this Example, the inventors tested the use of hydrolyzed and functionalized starch compounds for their printability, in particular by an extrusion method, and in particular for their ability to print instant release dosage forms.

The inventors prepared filaments from various formulations, as shown in Table 2.

**[Table 2]**

| Ref | F1 | F2 | F3 | F4 | F5 | F6 | F7 |
|---|---|---|---|---|---|---|---|
| H-HP1 (LYCOAT^{®} RS720) | 42.4% | / | / | / | / | / | / |
| X-X1 (PREGEFLO^{®} L100G) | / | 42.4% | / | / | / | / | / |
| X-X2 (LYCATAB^{®} PGS) | / | / | 42.4% | / | / | / | / |
| X-HP1 (LYCOAT^{®} NG720) | / | / | / | 42.4% | / | / | / |
| H-X1 (GLUCIDEX^{®} 1) | / | / | / | / | 42.4% | / | / |
| H-X2 (GLUCIDEX^{®} 2) | / | / | / | / | / | 42.4% | / |
| H-X3 (KLEPTOSE^{®} Linecaps) | / | / | / | / | / | / | 42.4% |
| Active ingredient (diprophylline) | 20% | 20% | 20% | 20% | 20% | 20% | 20% |
| Mannitol | 7.2% | 7.2% | 7.2% | 7.2% | 7.2% | 7.2% | 7.2% |
| Sorbitol | 28.8% | 28.8% | 28.8% | 28.8% | 28.8% | 28.8% | 28.8% |
| Stearic acid | 1.6% | 1.6% | 1.6% | 1.6% | 1.6% | 1.6% | 1.6% |

In this tables, percentages are expressed by weight, with respect to the total weight of the formulation (powder mix used to prepare the filaments).

Ingredients were sieved through a 500µm sieve and blending was done at 40rpm. For preparing filaments, the blend was then passed through a twin-screw extruder, which consist of 4 zones (from feeding zone, mixing, kneading and exit): the temperature for each zone was: 120°C, 160°C, 160°C and 120°C.

Printing was done using an Ultimaker S5 printer with printhead BB core (mainly for water soluble materials) at 180°C, speed is at 4mm/s, and 0.065mm as print height. Tablets to be printed were cylindrical tablets of 10mm diameter and 5mm height.

Only the filaments obtained from formulation F1 could achieve a continuous printing. The printing took about 1 hour for 1 tablet. Filaments obtained from formulations F2, F3, F5 and F6 were too brittle to be printed. As for formulations F4 and F7, the obtaining of filaments was impossible: formulation F4 was not extrudable, and the extrudate obtained with formulation F7 was liquid.

The tablets (at least 6 tablets) obtained from formulation F1 were tested for their instant release properties according the guidance as provided by US FDA (Dissolution Testing and Acceptance Criteria for Immediate-Release Solid Oral Dosage Form Drug Products Containing High Solubility Drug Substances; Guidance for Industry ; August 2018, Biopharmaceutics), using USP apparatus 2 (Paddle method), in 900mL of simulated gastric fluid media for 30 minutes. The drug concentration was accessed using UV spectrometry machine.

The release profile obtained is shown in Figure 2. These results show that hydrolyzed and functionalized starch compounds according to the disclosure, in particular hydrolyzed etherified and/or esterified starch compounds, can be used in 3DP, in particular in particular using HME, in particular by FDM. These results also show that hydrolyzed and functionalized starch compounds according to the disclosure can be used for preparing instant release dosage forms by 3DP.

### C. Example 2 - Preparation of solubility-enhanced tablets comprising a water-insoluble active ingredient, having controlled release properties, by FDM

In this Example, the inventors tested the use of hydrolyzed and functionalized starch compounds for their printability, and in particular for their ability to print dosage forms comprising water-insoluble active ingredients.

The inventors prepared filaments from the formulation as shown in Table 3.

**[Table 3]**

| Ref | F8 |
|---|---|
| H-HP2 (LYCOAT^{®} RS780) | 26% |
| H-OS1 (CLEARGUM^{®} CO03) | 24% |
| Active ingredient (itraconazole) | 10% |
| Sorbitol | 40% |

In this tables, percentages are expressed by weight, with respect to the total weight of the formulation (powder mix used to prepare the filaments).

Ingredients were sieved through a 500µm sieve and blending was done at 40rpm. For preparing the filament(s), the blend was then passed through a twin-screw extruder, which consist of 4 zones (from feeding zone, mixing, kneading and exit): the temperature for each zone was: 120°C, 160°C, 160°C and 100°C.

Printing was done using an Ultimaker S5 printer with printhead BB core (mainly for water soluble materials) at 180°C, speed is at 4mm/s, and 0.065mm as print height. Tablets to be printed were cylindrical tablets of 10mm diameter and 5mm height.

The filaments obtained from formulation F8 could achieve a continuous printing. Furthermore, it should be noted that the inventors performed the printing using filaments from different batches, and shown similar release pattern and drug contents, reflecting the good content uniformity of the filaments.

The tablets (at least 6 tablets) obtained from formulation F8 were tested for their release properties in the same manner as described in previous Example B, except that the duration was 8 hours. The drug concentration was accessed using HPLC, with acetonitrile and water at ratio of 60:40 as mobile phase.

For comparison, tablets by direct compression were also prepared from formulation F8, having the same shape as the ones obtained by 3DP. The inventors used a 10mm flat face bevel edge (FFBE) tablet punch, compressed the powder blends (500mg) to 4mm with compression time of around 1000ms. Once removed from the machine (STYL'One tablet machine), the cylindrical tablets were measured and heights, diameter and weights were recorded at around 5mm, 10mm and 500mg respectively.

The release profiles obtained with the 3D printed dosage forms and with the dosage forms obtained by standard direct compression are shown in Figure 3. These results show that hydrolyzed and functionalized starch compounds according to the disclosure, in particular hydrolyzed etherified and/or esterified starch compounds, can also be used for preparing dosage forms containing water-insoluble active ingredients by 3DP. The release is greatly improved, as compared to when standard compression process is used for making the tablets. After 8 hours, 60% dry weight of active ingredient was released for the 3D printed tablet, whereas, 5% release is obtained with the tablets obtained by standard compression. These results show that hydrolyzed and functionalized starch compounds according to the disclosure, in particular hydrolyzed etherified and/or esterified starch compounds, can advantageously be used for preparing solubility-enhanced dosage forms, and/or for preparing controlled release dosage forms.

### D. Example 3 - Preparation of a modified release tablet comprising a water-soluble active ingredient by PAM

In this Example, the inventors tested the use of hydrolyzed and functionalized starch compounds for their printability, and in particular for their ability to print modified release dosage forms comprising water-soluble active ingredients. Filaments were prepared from these formulations and then tablets were prepared by PAM. The tablets thus obtained were tested for their release profile.

The inventors prepared a gel-mass as a printable material having the formulation as shown in Table 4.

**[Table 4]**

| Ref | F9 | |
|---|---|---|
| | Powder mix | Gel-mass |
| H-HP2 (LYCOAT^{®} RS780) | 12.3% | 6.75% |
| H-OS1 (CLEARGUM^{®} CO03) | 36.9% | 20.25% |
| Active ingredient (diprophylline) | 9.8% | 5.5% |
| crosslinked pregelatinized and cold water-soluble potato starch (PREGEFLO^{®} PI10) | 20.5% | 11.25% |
| Microcrystalline cellulose | 20.5% | 11.25% |
| Solvent (water) | / | 45% |

In this tables, percentages are expressed by weight, with respect to the total weight of the formulation (powder mix for preparing the gel-mass and gel-mass).

The gel-mass was prepared as follow: the powder mix was blended manually for at least 10 minutes before adding into 90°C water under stirring condition. Mixtures were left cool-down under stirring condition for at least 4 hours before being transferred to 3mL printing cartridge. The nozzle used was 0.41mm (21 gauze). Printing pattern was cylinder with 10 mm diameter and 5 mm height. The printing parameters were as follow: Pressure 90-110 kPa; Speed 1 mm/s; Layer height 0.41 mm; First layer compensation 75%; Printer BloX. Printing density was varied (i.e. 50% and 80%).

The formed tablets were then vacuum dried to ensure complete drying.

The tablets (at least 6 tablets) obtained from formulation F9 were tested for their modified release properties according to the guidance as provided by the US FDA (General Methods; 32(2) Second Interim Revision Announcement: DISSOLUTION, <711> DISSOLUTION; 11/21/2016),using dissolution method A (pH transition method, simulated gastric fluid for 2 hours then change to simulated intestinal fluid for the remaining 10 hours).

The results are shown in Figure 4. After 30 minutes, 41% by dry weight of the active ingredient is released for the tablets printed with 50% density. After 30 minutes, 29% by dry weight of the active ingredient is released for the tablets printed with 80% density.

These results show that hydrolyzed and functionalized starch compounds according to the disclosure, in particular hydrolyzed etherified and/or esterified starch compounds, can be used in 3DP, in particular in particular using cold-extrusion, in particular by PAM. This could be useful for 3D bioprinting. These results also show that hydrolyzed and functionalized starch compounds according to the disclosure, in particular hydrolyzed etherified and/or esterified starch compounds, can be used for preparing modified release dosage forms by 3DP. By varying the printing density, it is advantageously possible to vary the release profile of the tablet.

## Claims

1. Use, for 3D printing, of a hydrolyzed and functionalized starch compound.

2. The use of claim 1, wherein said hydrolyzed and functionalized starch compound has a cold-water solubility equal to or higher than 30%, said percentage being expressed in dry weight of soluble starch with respect to the total weight of starch.

3. The use of any of claims 1 or 2, wherein said hydrolyzed and functionalized starch compound has a weight average molecular weight (Mw) lower than 10,000 kDa as determined by size-exclusion chromatography (HPSEC) attached to multiangle laser-light scattering (MALLS) and refractive index (RI) detectors.

4. The use of any of claims 1 to 3, wherein said hydrolyzed and functionalized starch compound has a viscosity lower than 10,000 mPa.s at 20°C, at a shear rate of 100 s⁻¹, as determined on an aqueous solution comprising 10% dry weight of said starch compound.

5. The use of any of claims 1 to 4, wherein said hydrolyzed and functionalized starch compound is selected from: a hydrolyzed starch compound functionalized with alkyl groups having 1 to 5 carbon atoms; an amphiphilic hydrolyzed and functionalized starch compound; or from a mixture thereof.

6. The use of claim 5, wherein said hydrolyzed starch compound functionalized with alkyl groups having 1 to 5 carbon atoms is hydrolyzed hydroxypropyl starch.

7. The use of claim 5, wherein said amphiphilic hydrolyzed and functionalized starch compound is octenylsuccinate starch.

8. The use of any of claims 1 to 7, wherein said hydrolyzed and functionalized starch compound is used in a printable material.

9. The use of claim 8, wherein said printable material is a powder, a filament, or a gel mass.

10. The use of any of claims 1 to 9, for the 3D printing of a dosage form.

11. The use of claim 10, for the 3D printing of a modified release dosage form, or of an instant release dosage form.

12. The use of any of claims 1 to 11, for 3D printing by a process using extrusion.

13. The use of claim 12, for 3D printing by a process using hot melt extrusion (HME) or cold-extrusion.

14. A process for the manufacture of a product by 3D printing, comprising the 3D printing of a printable material comprising a hydrolyzed and functionalized starch compound.

15. A filament for 3D printing, comprising a hydrolyzed and functionalized starch compound.
